(19) Europäisches Patentamt
European Patent Office
Office européen des brevets

(11) **EP 4 260 818 A1**

(12) **EUROPEAN PATENT APPLICATION**
published in accordance with Art. 153(4) EPC

(43) Date of publication:
**18.10.2023 Bulletin 2023/42**

(21) Application number: **21917316.8**

(22) Date of filing: **28.12.2021**

(51) International Patent Classification (IPC):
**A61B 17/12** (2006.01)  **A61F 2/90** (2013.01)
**A61F 2/97** (2013.01)  **A61F 2/966** (2013.01)

(52) Cooperative Patent Classification (CPC):
**A61F 2/07; A61F 2/86; A61F 2/88; A61F 2/966;**
A61F 2002/9665; A61F 2250/0071;
A61F 2250/0098

(86) International application number:
**PCT/CN2021/142230**

(87) International publication number:
**WO 2022/148277 (14.07.2022 Gazette 2022/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2021  CN 202110013051**

(71) Applicant: **MicroPort NeuroTech (Shanghai) Co., Ltd.**
**Shanghai 201318 (CN)**

(72) Inventors:
• ZHAO, Hanyi
 Shanghai 201318 (CN)
• ZHANG, Miao
 Shanghai 201318 (CN)
• TIAN, Hao
 Shanghai 201318 (CN)
• LIU, Ziang
 Shanghai 201318 (CN)
• WANG, Qinfen
 Shanghai 201318 (CN)

(74) Representative: **Marks & Clerk LLP**
**15 Fetter Lane**
**London EC4A 1BW (GB)**

(54) **VASCULAR IMPLANT AND MEDICAL DEVICE**

(57)    A vascular implant and a medical device. The vascular implant includes a tubular implant main segment (100, 400) braided from two or more interlaced wires (130, 132, 134, 136, 138) and having a first end (120, 420) and a second end (110, 410), which are located at two ends of g an axis of the implant main segment. The first end (120, 420) includes a plurality of looped-back rings (122, 124, 422, 424). The second end (110) in-cludes a plurality of first joint portions (112), each of which is a non-invasive joint portion formed by joining at least two of the wires (130, 132, 134, 136, 138). At least one of the two or more wires (130, 132, 134, 136, 138) in-cludes core and a jacket surrounding the core. The ends of the vascular implant will cause less damage to the wall of a blood vessel, and the implant provides improved stability and radiopacity.

Fig. 1

EP 4 260 818 A1

**Description**

**TECHNICAL FIELD**

The present invention relates to the field of medical devices and, in particular, to a vascular implant and a medical device.

**BACKGROUND**

Minimally invasive intervention provides a treatment for vascular aneurysms. It usually involves delivery of a vascular implant such as, but not limited to, a stent, a coil or an aneurysm occlusion device, to a lesion site in a blood vessel using a delivery device. After that, a delivery rod is used to release the vascular implant while maintaining an intended configuration of the implant. In this way, the implant can provide a therapeutic effect by dilating the blood vessel or occluding an aneurysm therein.

As one type of such vascular implants, self-expanding braided stents have found extensive use thanks to their good delivery performance. As conventional self-expanding braided stents are usually braided from many wires, there are typically multiple wires having sharp ends at their end portions. Therefore, they are associated with a risk of causing damage to blood vessels during use. Moreover, the wires are typically loose, making the stents insufficient in structural stability and prone to the issues of deformation and lumen collapse when deployed in blood vessels.

Therefore, there is a need for a novel vascular implant which overcomes at least the above disadvantages.

**SUMMARY OF THE INVENTION**

The present invention seeks to provide a stent structure for a vascular implant, which will cause less damage to a blood vessel at its ends and has enhanced stability.

To this end, the present invention provides a vascular implant comprising a tubular implant main segment braided from two or more interlaced wires, wherein the implant main segment has a first end and a second end that are located at two ends of an axis of the implant main segment.

The first end comprises a plurality of looped-back rings, at least one of which is provided with a radiopaque marker at its circumference.

The second end comprises a plurality of first joint portions, each of which is a non-invasive joint portion formed by joining at least two of the wires.

Additionally, the implant main segment provides a radial support force in a blood vessel, which is in the numerical range of 0.03-0.12 N/mm and satisfies the following formula:

$$F = aNd^2 \, \theta^2$$

wherein $a$ is a constant, N is the number of wires, $d$ is a diameter of the wires, and $\theta$ is an angle at which the wires are interlaced in a middle section of the implant main segment.

In some embodiments, the present invention provides another vascular implant comprising a tubular implant main segment braided from two or more interlaced wires, wherein the implant main segment has a first end and a second end that are located at two ends of an axis of the implant main segment.

The first end comprises a plurality of looped-back rings, at least one of which is provided with a radiopaque marker at its circumference.

The second end comprises a plurality of first joint portions, each of which is a non-invasive joint portion formed by joining at least two of the wires.

At least one of the two or more wires comprises a core and a jacket surrounding the core. The core is made of one of platinum, iridium, gold, silver, tantalum and tungsten or an alloy thereof, and the jacket is made of one or more of a nickel-titanium alloy, nitinol, stainless steel, a cobalt-chromium alloy and a nickel-cobalt alloy. A cross-sectional area of the core accounts for 20%-35% of a total cross-sectional area of the wire.

In some embodiments, the present invention provides yet another vascular implant comprising a tubular implant main segment braided from two or more interlaced wires, wherein the implant main segment has a first end and a second end that are located at two ends of an axis of the implant main segment.

The first end comprises a plurality of looped-back rings.

The second end comprises a plurality of first joint portions, each of which is a non-invasive joint portion formed by joining at least two of the wires.

At least one of the two or more wires comprises a core and a jacket surrounding the core. The core is made of one of platinum, iridium, gold, silver, tantalum and tungsten or an alloy thereof, and the jacket is made of one or more of a nickel-titanium alloy, nitinol, stainless steel, a cobalt-chromium alloy and a nickel-cobalt alloy.

Optionally, the first end may comprise a plurality of first looped-back rings and a plurality of second looped-back rings, which are alternately arranged in a circumferential direction.

Optionally, the first and second looped-back rings may have equal lengths along the axis of the tubular implant main segment, wherein the plurality of first looped-back rings or the plurality of second looped-back rings are provided with at least one radiopaque marker at their circumference. Specifically, at least one of the plurality of first looped-back rings and the plurality of second looped-back rings may be provided with at least one radiopaque marker in its circumferential direction.

Optionally, the first looped-back rings may have a length along the axis of the tubular implant main segment, which is greater than a length of the second looped-back rings along the axis of the tubular implant main segment, wherein the plurality of first looped-back rings are provided with at least one radiopaque marker at their circumference. Specifically, at least one of the plurality of first looped-back rings may be provided with at least one radiopaque marker in its circumferential direction.

Optionally, the second end may further comprise a plurality of third looped-back rings, wherein the first and third looped-back rings are alternately arranged in a circumferential direction.

Optionally, the first joint portions may have a length along the axis of the tubular implant main segment, which is greater than a length of the third looped-back rings along the axis of the tubular implant main segment.

Optionally, the plurality of first joint portions and the plurality of first looped-back rings may be arranged in symmetry at the opposite ends of the vascular implant. Additionally or alternatively, the plurality of third looped-back rings and the plurality of second looped-back rings may be arranged in symmetry at the opposite ends of the vascular implant.

Optionally, the looped-back ring may be formed by looping back one of the wires into an arc, wherein the arc is a semicircular, a semi-elliptic or a semicircle-like structure.

Optionally, the looped-back ring may be formed by looping back one of the wires and bonding or welding it to another one of the wires.

Optionally, the looped-back ring may be formed by looping back one of the wires and bonding or welding it to another one of the wires via a radiopaque marker, wherein the radiopaque marker is a radiopaque spring or a radiopaque sleeve.

Optionally, the looped-back ring may be formed by coiling an end of the looped-back wire to form a spring-like radiopaque marker and then bonding or welding it to another wire. Optionally, two wires used to form the first joint portion are juxtaposed or twisted and then welded, wherein an end portion of the first joint portion is a smooth welded joint.

Optionally, two wires used to form the first joint portion are juxtaposed or twisted and then bonded or welded via a tubular spring or a cylindrical tube, wherein an end portion of the tubular spring or the cylindrical tube is bonded or welded with a smooth doom-shaped end cap.

Optionally, the implant main segment may have a mesh structure with diamond-shaped mesh openings, which is braided from 12-32 interlaced wires, wherein 10-75 intersections per inch may be formed by the wires along an axial direction.

Optionally, the implant main segment may have a mesh structure with diamond-shaped mesh openings, which is braided from 16-24 interlaced wires, wherein 30-55 intersections per inch are formed by the wires along an axial direction.

Optionally, metal coverage of the implant main segment may range from 8% to 25%.

Optionally, the cross-sectional area of the core may account for 15%-40% of the total cross-sectional area of the wire.

Optionally, the cross-sectional area of the core may account for 20%-35% of the total cross-sectional area of the wire.

To the above end, the present invention also provides a medical device comprising any of the above vascular implants and a delivery rod. The delivery rod includes a second joint portion configured for detachable snap engagement with the first joint portions of the vascular implant.

Optionally, the medical device may further comprise a lumen for receiving the delivery rod and the second end of the vascular implant therein, wherein when the second end of the vascular implant and the delivery rod are received within the lumen, the second end of the vascular implant is constrained by the lumen therefore remains in snap engagement with the second joint portion, thereby axially locking the vascular implant to the delivery rod, or wherein when the second end of the vascular implant is released from the lumen, the second end of the vascular implant is no longer constrained by the lumen and therefore detachable from the second joint portion, thereby axially unlocking the vascular implant from the delivery rod.

In summary, the vascular implants and the medical device provided in the present invention have the advantages as follows.

First, the structures of the vascular implants are designed with smooth end closure structures, which can reduce the risk of damage being caused by randomly pointing or sharp end portions of the wires to the wall of a blood vessel, which may lead to inflammatory response to the implant. In this way, higher surgical safety and better therapeutic outcomes can be obtained.

Second, the smooth closed structures provided at the ends of the vascular implants impart improved stability to the stents, enabling them to be better supported within a blood vessel. As a result, they are able to desirably maintain their lumen, resist to deformation or collapse, provide support to a diseased blood vessel and deliver very good therapeutic effects.

Third, the wires in the vascular implants are made of a radiopaque material, imparting better radiopacity to the stents and increased safety to surgical procedures using them. Moreover, the wires may be of different materials and/or sizes, allowing flexible adjustability of the stents' radial support force and flexibility and making them suitable for use in more scenarios and a wider range of applications.

Fourth, in the medical device, the vascular implant can be proximally coupled to the delivery rod with a snap engagement feature. Preferably, the proximal end of the vascular implant includes two or more first joint portions, which are spaced apart circumferentially around the vascular implant. Moreover, snap engagement members of the first joint portions are staggered from one another along the axis of the vascular implant. This allows the snap engagement members to be crimped at different circumferences along the axial length. In this way, the vascular implant and the delivery device are allowed to have reduced sizes in a compressed configuration of the vascular implant, and the vascular implant can be delivered within a smaller delivery device. This not only enables the delivery device to access a target site within a farther or narrower blood vessel, but also allows a catheter to be multiplexed in two systems, resulting in time savings for surgery.

## BRIEF DESCRIPTION OF THE DRAWINGS

Fig. 1 is a perspective view of a braided stent according to an embodiment of the present invention;
Fig. 2 is a front view of the braided stent of Fig. 1;
Fig. 3 is a right view of the braided stent of Fig. 1;
Fig. 4 is a partial front view of a braided stent according to another embodiment of the present invention;
Fig. 5 is a right view of the braided stent of Fig. 4;
Figs. 6A to 6C schematically illustrate respective three structural embodiments of first looped-back rings in the braided stent of Fig. 1;
Figs. 7A to 7D schematically illustrate respective four structural embodiments of first joint portions in the braided stent of Fig. 1;
Fig. 8 schematically illustrates a delivery rod being coupled to a braided stent according to an embodiment of the present invention.

reference numerals in drawings:

10 - stent; 100 - implant main segment; 110 - second end; 112 - first joint portion; 114 - third looped-back ring; 120 - first end; 122 - first looped-back ring; 124 - second looped-back ring; 126 - radiopaque marker; 130, 132, 134, 136, 138 - wires; 137 - cylindrical tube; 400 - implant main segment; 410 - second end; 420 - first end; 422 - first looped-back ring; 424 - second looped-back ring; 20 - medical device; 300 - delivery rod; 320 - second joint portion; 340 - delivery sheath; 360 - lumen.

## DETAILED DESCRIPTION

Objects, advantages and features of the present invention will become more apparent upon reading the following more detailed description of the present invention, which is made with reference to the accompanying drawings. Note that the figures are provided in a very simplified form not necessarily drawn to exact scale and for the only purpose of facilitating easy and clear description of the embodiments. Whenever there is no conflict, the embodiments disclosed herein and features thereof can be combined with one another.

As used herein, the singular forms "a", "an" and "the" include plural referents, and the term "plurality" is employed in the sense of "two or more", unless the context clearly dictates otherwise. As used herein, the term "or" is generally employed in the sense of "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context. Throughout the several views, similar numerals indicate similar elements.

In the following, for ease of description, the terms "distal end" and "proximal end" are used. The term "proximal end" refers to an end of a medical device closer to an operator who is operating the device, and the term "distal end" refers to an end of the medical device away from the operator. The following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the invention.

In principle, the present invention seeks to provide a vascular implant, in particular a braided stent for use in the treatment of cerebrovascular disease. The vascular implant can be delivered to a target site by a delivery device to treat an intracranial aneurysm or other vascular disease. It will be understood that the vascular implant may also be used to treat an intracranial aneurysm or a non-intracranial aneurysm, dilate a blood vessel, capture a thrombus in a blood vessel, or treat another luminal lesion.

The present invention provides a vascular implant including a tubular implant main segment braided from two or more interlaced wires. The implant main segment has a first end and a second end, which are located at two ends of an axis of the implant main segment.

The first end includes a plurality of looped-back rings, at least one of which is provided with a radiopaque marker at its circumference (in its circumferential direction).

The second end includes a plurality of first joint portions, each of which is a non-invasive joint portion formed by joining at least two wires.

When deployed in a blood vessel, the implant main segment can provide radial support force in the numerical range of 0.03-0.12 N/mm. The radial support force satisfies the following formula:

$$F=aNd^2\theta^2$$

wherein *a* is a constant, N is the number of wires, *d is a* diameter of the wires, and $\theta$ is an angle at which the wires are interlaced in a middle section of the implant main segment.

In some embodiments, the present invention provides another vascular implant including a tubular implant main segment braided from two or more interlaced wires. The implant main segment has a first end and a second end, which are located at two ends of an axis of the implant main segment.

The first end includes a plurality of looped-back rings, at least one of which is provided with a radiopaque marker at its circumference (in its circumferential direction).

The second end includes a plurality of first joint portions, each of which is a non-invasive joint portion formed by joining at least two wires.

At least one of the two or more wires includes a core and a jacket surrounding the core. The core is made of one of platinum, iridium, gold, silver, tantalum and tungsten, or an alloy thereof. The jacket is made of one or more of a nickel-titanium alloy, nitinol, stainless steel, a cobalt-chromium alloy and a nickel-cobalt alloy. For each wire including a core and a jacket surrounding the core, a cross-sectional area of the core accounts for 20%-35% of a total cross-sectional area of the wire.

In some embodiments, the present invention provides yet another vascular implant including a tubular implant main segment braided from two or more interlaced wires. The implant main segment has a first end and a second end, which are located at two ends of the axis of the implant main segment

The first end includes a plurality of looped-back rings.

The second end includes a plurality of first joint portions, each of which is a non-invasive joint portion formed by joining at least two wires.

At least one of the two or more wires includes a core and a jacket surrounding the core. The core is made of one of platinum, iridium, gold, silver, tantalum and tungsten, or an alloy thereof. The jacket is made of one or more of a nickel-titanium alloy, nitinol, stainless steel, a cobalt-chromium alloy and a nickel-cobalt alloy.

The vascular implants and a therapeutic device provided in the present invention will be further described below with reference to the accompanying drawings and some embodiments.

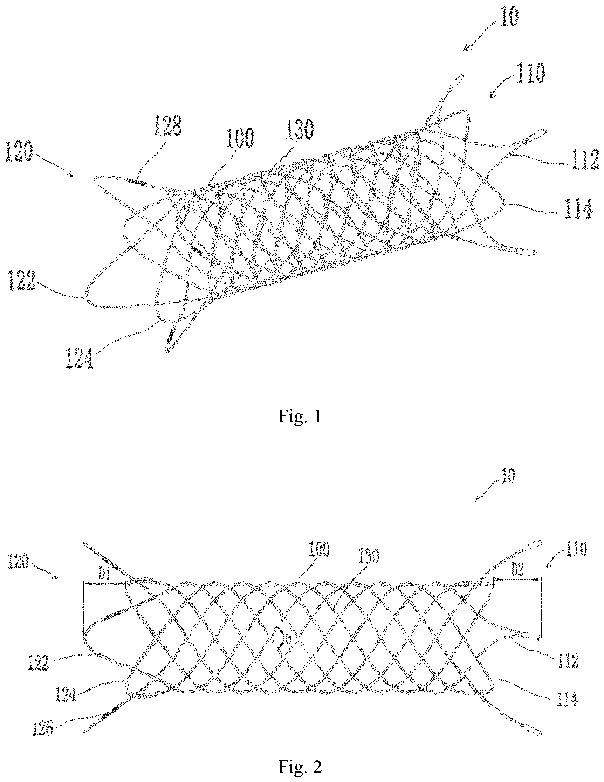

As shown in Figs. 1 and 2, any of the above vascular implants may be a braided stent 10 including a tubular implant main segment 100 braided from two or more interlaced wires 130. At least one of the two or more wires 130 includes a core and a jacket surrounding the core. The core is made of one of radiopaque materials including platinum, iridium, gold, silver, tantalum and tungsten, or an alloy thereof. The jacket is made of one or more of a nickel-titanium alloy, nitinol, stainless steel, a cobalt-chromium alloy and a nickel-cobalt alloy. The implant main segment 100 may have an axial length of 5-30 mm.

In some embodiments, the implant main segment 100 has a mesh structure with diamond-shaped mesh openings, which is braided from 12-32 interlaced wires 130 with 10-75 intersections per inch formed along the axial direction. It will be understood that, in a resting (or non-compressed) configuration of the braided stent 10, the number of intersections of the wires may vary from 10 to 75 per inch along the axial direction, depending on design dimensions of the stent. However, when the braided stent 10 is in a compressed configuration, depending on dimensions of the blood vessel portion where it is located (i.e., depending on how much it is compressed), the number of intersections of the wires may be reduced to as few as 10 per inch along the axial direction. In a preferred embodiment, the implant main segment 100 is a mesh structure with diamond-shaped mesh openings, which is braided from 16-24, such as 16, 20 or 24, interlaced wires 130 with 30-55 intersections per inch formed along the axial direction. Metal coverage of the implant main segment 100 consisting of those wires may range from 8% to 25% (the metal coverage is defined as a percentage of a total area occupied by the metal wires in a total surface area of the implant).

It will be understood that the stent may be required to provide different radial support force for different blood vessels in the same or different patients, or for different locations of the same blood vessel. In embodiments of the present invention, the radial support force that the implant main segment 100 provides in a blood vessel is in the range of 0.03-0.12 N/mm and satisfies the following formula:

$$F=aNd^2\theta^2$$

wherein *a* is a constant, N is the number of wires, *d is a* diameter of the wires, and $\theta$ is an angle at which the wires are interlaced in a middle section of the implant main segment. The constant *a* is related to parameters such as the properties of the wire material. The middle section refers to an intermediate effective portion of the implant other than its flared end portions.

In some embodiments, as shown in Fig. 2, the angle $\theta$ that the wires 130 are interlaced at in the middle section of the implant main segment 100 may range from 70° to 160°. In preferred embodiments, the angle is in the range of 120-160°.

In some embodiments, braiding of the stent may be designed according to the above formula to meet various stent radial support force requirements of different blood vessels. For example, the number, diameter and/or angle of wires may be varied to expand the stent's scope of application.

In some embodiments, all the wires 130 in the implant main segment 100 are composite wires consisting of a core and a jacket surrounding the core. The composite wires 130 may have a diameter in the range of from 0.0016 inches to 0.0024 inches, and a ratio of a cross-sectional area of each core in a total cross-sectional area of the specific wire 130 ranges from 15%-40%, preferably, 20%-35%. The radiopaque material of the wires imparts better radiopacity to the stent, which enhances surgical safety. In some other embodiments, some wires in the implant main segment 100 are composite wires consisting of a core and a jacket surrounding the core, while the remaining ones may be made of a different material and/or have a different size. For example, they may be composite wires with a diameter in a different range, or made of one or more of a nickel-titanium alloy, nitinol, stainless steel, a cobalt-chromium alloy and a nickel-cobalt alloy. These wires made of a different material and/or with a different size can reduce the cost of the stent and enables it to be applied in more scenarios and a wider range of applications.

As shown in Figs. 1 and 2, the implant main segment 100 has a first end 120 and a second end 110, which are located at two ends of an axis of the implant main segment 100. In some embodiments, the first end 120 is a distal end of the implant main segment 100, and the second end 110 is a proximal end of the implant main segment 100. The first end 120 includes a plurality of first looped-back rings 122 and a plurality of second looped-back rings 124. The first looped-back rings 122 and the second looped-back rings 124 are alternately arranged in a circumferential direction. In other embodiments, the first looped-back rings 122 and the second looped-back rings 124 may be circumferentially arranged either in a regular or irregular pattern. For example, there may be only two first looped-back rings which are arranged in symmetry, and all the other looped-back rings are second looped-back rings.

In some embodiments, the first end 120 and/or the second end 110 of the implant main segment 100 may be flared, and an angle formed of the first end 120 and/or the second end 110 with respect to an axis of the implant main segment 100 ranges from 15-45°.

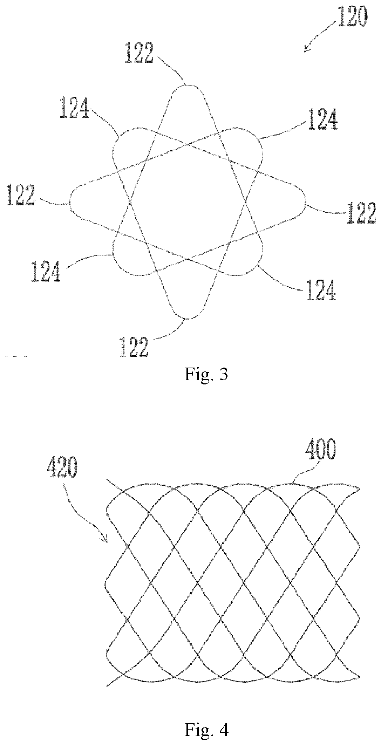

With continued reference to Fig. 3, the first end 120 may include 4 first looped-back rings 122 and 4 second looped-back rings 124, which are alternately arranged in a circumferential direction. The first looped-back rings 122 may have a length along the axis of the tubular implant main segment 100, which is greater than a length of the second looped-back rings 124 along the axis of the tubular implant main segment 100. The difference between the lengths of the first looped-back rings 122 and the second looped-back rings along the axis of the tubular implant main segment 100 is denoted as D1 and may range from 0.5 mm to 2 mm. Axially staggering the first looped-back rings 122 from the second looped-back rings 124 allows the vascular implant to be compressed into a smaller size and thus delivered within a smaller delivery device. In some embodiments, at least one of the 4 first looped-back rings 122 is provided with at least one radiopaque marker 126 at its circumference (in its circumferential direction). In order not to cause an increased axial size of the braided stent 10, and not to cause damage to a blood vessel, the radiopaque marker may be not provided at an apical location of the first looped-back ring 122. In the embodiment shown in Fig. 2, three of the 4 first looped-back rings 122 are each provided with a radiopaque marker 126. These radiopaque markers 126 can make the stent more radiopaque during a surgical procedure and thus help a surgeon to more accurately locate the stent, resulting in higher surgical safety. Providing three radiopaque markers can ensure good radiopacity while allowing the stent to be compressed into a smaller radial size, which enables the stent to be delivered within a smaller delivery system.

In some other embodiments, 2, 6, 8 or more first looped-back rings 122 and second looped-back rings 124 may be included. The radiopaque markers 126 may be also provided on the second looped-back rings 124, and the number of the radiopaque marker may be at least one.

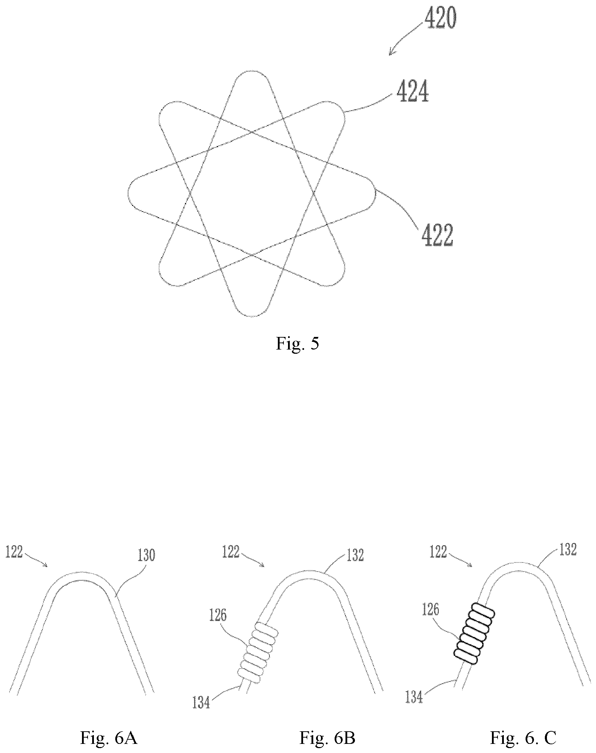

As shown in Figs. 4 and 5, in some embodiments, the first end 420 of the implant main segment 400 includes 4 first looped-back rings 422 and 4 second looped-back rings 424, which are arranged circumferentially with an equidistant space. Moreover, the first looped-back rings 422 and the second looped-back rings 424 have equal lengths along the axis of the tubular implant main segment 400. In some embodiments, the 4 first looped-back rings 422 or the 4 second looped-back rings 424 are provided at their circumference with at least one radiopaque marker (not shown), e.g., 2, 3, 4, 6 or 8 radiopaque markers. In some other embodiments, the number of each of the first looped-back rings 422 and second looped-back rings 424 may be 2, 6, 8 or more.

Figs. 6A to 6C schematically illustrate respective three structural embodiments of the first looped-back rings 122 of Fig. 2. In a single design of the braided stent 10, a single first looped-back ring structure or two or more of the first looped-back ring structures may be used. In some embodiments, the second looped-back rings 124 may also have the same structure(s).

As shown in Fig. 6A, a first looped-back ring 122 is formed from one of the wires 130 having a portion thereof looped back to form an arc, wherein the arc is a semicircular, a semi-elliptic or a semicircle-like structure.

As shown in Fig. 6B, a first looped-back ring 122 is formed from one of the wires 132 having a portion thereof looped back and directly bonded or welded to another one of the wires 134. Specifically, one end of the looped-back wire 132 is coiled into a spring serving as a radiopaque marker 126 and then bonded or welded to the other wire 134.

As shown in Fig. 6C, a first looped-back ring 122 is formed from one of the wires 132 having a portion thereof looped back and bonded or welded to another one of the wires 134 through a radiopaque marker 126, wherein the radiopaque marker 126 may be implemented as either a radiopaque marker or a radiopaque sleeve.

The braided stent is provided at its distal end with smooth closed structures. These can reduce the risk of damage being caused by randomly pointing or shape end portions of the wires to the wall of a blood vessel, which may lead to inflammatory response to the implant. In this way, higher surgical safety and better therapeutic outcomes can be obtained.

With continued reference to Figs. 1 and 2, the second end 110 of the implant main segment 100 includes a plurality of first joint portions 112, each of which is a non-invasive joint portion formed by joining at least two wires 130. Approaches that can be used to join the wires 130 together may include, but are not limited to, twisting, adhesive bonding and welding.

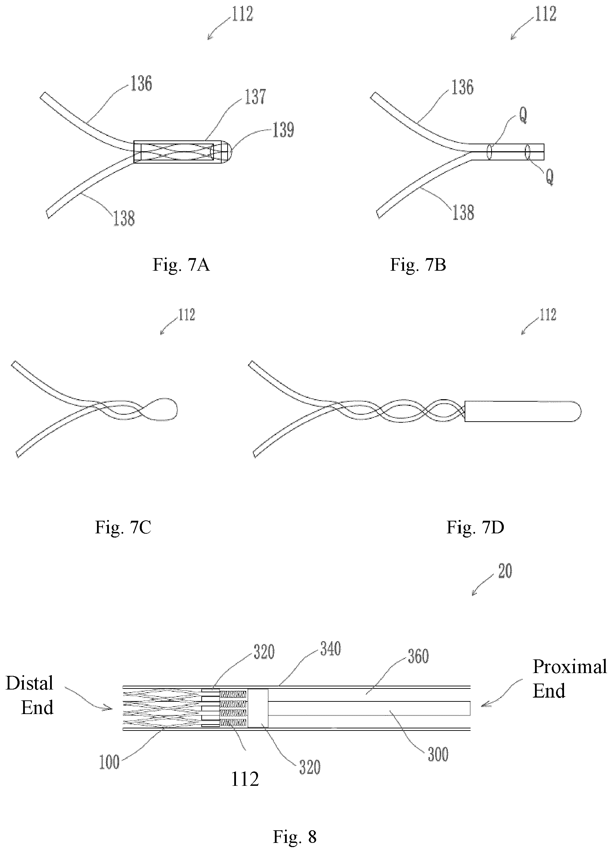

Figs. 7A to 7D schematically illustrate respective four structural embodiments of the first joint portions 112 of Fig. 2. The second end 110 may employ either one or two or more selected from the four structures. As shown in Figs. 7A to 7D, each first joint portion 112 consists of two end portions of respective wires 130 which are twisted or bundled together to form a closed end structure without a curved transition. That is, the two end portions of wires 130 form an angle of almost zero degrees. Such closed ends without any curved transition enable better anchoring of the braided stent within a blood vessel and more effective coverage of a target lesion therein.

In the embodiment shown in Fig. 7A, a first joint portion 112 is formed by twisting and coupling (end portions of) two wires 136 and 138 together, disposing a main part of a cylindrical tube 137 over the twisted end portions and bonding or welding them together. Laser welding may be applied to an end of the cylindrical tube 137 to form there a smooth, dome-shaped end closure structure which serves as a non-invasive joint portion. The cylindrical tube 137 may be replaced with a tubular spring. In some embodiments, the cylindrical tube 137 may be made of a noble metal that is opaque to X-rays, or of stainless steel. In order to ensure welding quality and stability of the stent structure, the closed end is preferred to be a proximal end closed with a cut metal ring.

In the embodiment shown in Fig. 7B, a first joint portion 112 is formed by juxtaposing (end portions of) two wires 136 and 138, and welding locations Q are formed at multiple positions. In some embodiments, after the wires are welded together, a cylindrical tube or tubular spring (not shown) may be disposed over them, followed by the application of laser welding to an end of the cylindrical tube or tubular spring, thereby forming a smooth, dome-shaped end closure structure serving as a non-invasive joint portion.

In the embodiment shown in Fig. 7C, a first joint portion 112 is formed by twisting (end portions of) two wires 136 and 138 together and applying laser welding to an end joint thereof to form there a smooth, dome-shaped end closure structure serving as a non-invasive joint portion.

The embodiment shown in Fig. 7D is similar to the embodiment shown in Fig. 7A, except that the twisted portions of the two wires 136, 138 are at least partially not received in the cylindrical tube 137. All the other features of this embodiment are shared with the embodiment shown in Fig. 7A and, therefore, need not be described in further detail herein.

In some embodiments, the second end 110 of the implant main segment 100 further includes a plurality of third looped-back rings 114, each of which may be a looped-back ring of a single wire. The first joint portions 112 and the third looped-back rings 114 may be alternately arranged in a circumferential direction. The number of the first joint portions 112 and/or third looped-back rings 114 may be 2, 3, 4, 6 or 8, which is not limited thereto.

In some embodiments, the first joint portions 112 may have a length along the axis of the tubular implant main segment 100, which is greater than or equal to a length of the third looped-back rings 114 along the axis of the tubular implant main segment 100. The difference between the lengths of the first joint portions 112 and the third looped-back rings 114 along the axis of the tubular implant main segment 100 is denoted as D2 and may range from 0.5 mm to 2 mm. Axially staggering the first joint portions 112 from the third looped-back rings 114 allows the vascular implant to be compressed into a smaller size and thus delivered within a smaller delivery device.

In some embodiments, the first looped-back rings 122 of the first end 120 and the first joint portions 112 of the second end 110 may be arranged in symmetry (with respect to a line normally bisecting the braided stent along a radial direction thereof) at (axially) opposite ends of the braided stent 10. That is, apices of the first joint portions 112 and respective apices of the first looped-back rings 122 are located on lines substantially parallel to an axial center line of the braided stent 10. This can facilitate determining how the stent is expanded and improve its safety of use.

In another aspect, the present invention also provides: a medical device including one of the above vascular implants; and a delivery rod including a second joint portion for detachable snap engagement with the first joint portions of the vascular implant.

Optionally, the medical device may further include a lumen for receiving the delivery rod and the second end of the vascular implant. When the second end of the vascular implant and the delivery rod are received in the lumen, the second end of the vascular implant will be constrained by the lumen so as to remain in snap engagement with the second joint portion, thereby axially locking the vascular implant to the delivery rod. When the second end of the vascular implant is released from the lumen, it will not be constrained by the lumen any longer and thus detachable from the second joint portion, and accordingly the vascular implant can be axially unlocked from the delivery rod.

Fig. 8 schematically illustrates the delivery rod and the braided stent, which are coupled together, according to an embodiment of the present invention. Referring to Figs. 2 and 8, in addition to the braided stent 10 and the delivery rod 300, the medical device 20 may further include a delivery sheath 340. The second end 110 of the implant main segment 100 comprises the first joint portions 112 which are configured for: snap engagement with the delivery rod 300 when constrained by the lumen 360 in the delivery sheath 340; or disengage from the delivery rod 300 when not constrained by the lumen 360. The delivery rod 300 may include at least one second joint portion 320. In the illustrated embodiment, the delivery rod 300 includes two second joint portions 320, which are spaced apart along an axis of a body of the delivery rod 300 at a distance configured to allow snap engagement of at least one of the first joint portions 112 therewith.

In specific implementations, when the second end 110 of the braided stent 10 and the delivery rod 300 are received within the lumen 360, the second end 110 of the braided stent 10 is constrained by the lumen 360 so as to maintain the second joint portions 320 in snap engagement with the first joint portions 112 and thereby axially lock the braided stent 10 to the delivery rod 300. As a result of proximal movement of the delivery sheath 340 relative to the delivery rod 300, the second end 110 of the braided stent 10 and the second joint portion 320 will not be radially constrained any longer. Because of the self-expanding properties of the braided stent 10, the first joint portions 112 of the braided stent 10 will radially move (outwardly) away from the second joint portions 320, resulting in disengagement and detachment of the second end 110 of the braided stent 10 from the delivery rod 300.

Compared with the prior art, the braided stent 10 is delivered within the delivery sheath 340 while being mechanically coupled to the delivery rod 300. This minimizes the chance of dislodgement of the braided stent 10 while it is being delivered and thus improves its delivery stability. Moreover, the snap engagement structure is simple and allows easy locking and unlocking. Additionally, this arrangement circumferentially differentiates axial locations of the first joint portions 112 (i.e., circumferentially stagger them from one another), allowing the braided stent 10 to be compressed into a smaller size and thereby making it deliverable within a smaller delivery sheath. This not only enables the stent to access a target site within a farther or narrower blood vessel, but also allows a catheter to be multiplexed in two systems, resulting in time savings for surgery.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A vascular implant, comprising a tubular implant main segment, wherein:

   the implant main segment is braided from two or more interlaced wires, wherein the implant main segment has a first end and a second end that are located at two ends of an axis of the implant main segment;
   wherein the first end comprises a plurality of looped-back rings;
   wherein the second end comprises a plurality of first joint portions, each of which is a non-invasive joint portion formed by joining at least two wires; and
   at least one of the two or more wires comprises a core and a jacket surrounding the core, wherein the core is made of one of platinum, iridium, gold, silver, tantalum and tungsten or an alloy thereof, and wherein the jacket is made of one or more of a nickel-titanium alloy, nitinol, stainless steel, a cobalt-chromium alloy and a nickel-cobalt alloy.

2. The vascular implant of claim 1, wherein at least one of the plurality of looped-back rings is provided with a radiopaque marker at a circumference thereof.

3. The vascular implant of claim 1 or 2, wherein for each wire comprising a core and a jacket surrounding the core, a cross-sectional area of the core accounts for 15%-40% of a total cross-sectional area of a corresponding wire.

4. The vascular implant of claim 1 or 2, wherein the implant main segment provides a radial support force in a blood vessel, wherein the radial support force is in a numerical range of 0.03-0.12 N/mm and satisfies the following formula:

$$F = aNd^2\,\theta^2$$

   wherein *a* is a constant, N is the number of wires, *d is* a diameter of the wire, and *θ* is an angle at which the wires are interlaced in a middle section of the implant main segment.

5. The vascular implant of any one of claims 1 to 4, wherein the first end comprises a plurality of first looped-back rings and a plurality of second looped-back rings, which are alternately arranged in a circumferential direction.

6. The vascular implant of claim 5, wherein the first and second looped-back rings have equal lengths along the axis of the tubular implant main segment, wherein the plurality of first looped-back rings or the plurality of second looped-back rings are provided with at least one radiopaque marker at a circumference thereof.

7. The vascular implant of claim 5, wherein a length of the first looped-back ring along the axis of the tubular implant main segment is greater than a length of the second looped-back ring along the axis of the tubular implant main segment, and wherein the plurality of first looped-back rings are provided with at least one radiopaque marker at a circumference thereof.

8. The vascular implant of any one of claims 6 to 7, wherein the second end further comprises a plurality of third looped-back rings, and wherein the first joint portions and third looped-back rings are alternately arranged in the circumferential direction.

9. The vascular implant of claim 8, wherein a length of the first joint portion along the axis of the tubular implant main segment is greater than a length of the third looped-back ring along the axis of the tubular implant main segment.

10. The vascular implant of claim 8, wherein the plurality of first joint portions and the plurality of first looped-back rings are arranged in symmetry at the opposite ends of the vascular implant; and/or wherein the plurality of third looped-back rings and the plurality of second looped-back rings are arranged in symmetry at the opposite ends of the vascular implant.

11. The vascular implant of any one of claims 1 to 4, wherein the looped-back ring is formed by looping back one of the wires into an arc, wherein the arc is a semicircular, a semi-elliptic or a semicircle-like structure.

12. The vascular implant of any one of claims 1 to 4, wherein the looped-back ring is formed by looping back one of the wires and bonding or welding it to another one of the wires.

13. The vascular implant of any one of claims 1 to 4, wherein the looped-back ring is formed by looping back one of the wires and bonding or welding it to another one of the wires via a radiopaque marker, and wherein the radiopaque marker is a radiopaque spring or a radiopaque sleeve.

14. The vascular implant of any one of claims 1 to 4, wherein the looped-back ring is formed by coiling an end of the looped-back wire to form a spring-like radiopaque marker and then bonded or welded to another wire.

15. The vascular implant of any one of claims 1 to 4, wherein two wires used to form the first joint portion are juxtaposed or twisted and then welded, wherein an end portion of the first joint portion is a smooth welded joint.

16. The vascular implant of any one of claims 1 to 4, wherein two wires used to form the first joint portion are juxtaposed or twisted and then bonded or welded via a tubular spring or a cylindrical tube, wherein an end portion of the tubular spring or the cylindrical tube is bonded or welded with a smooth doom-shaped end cap.

17. The vascular implant of any one of claims 1 to 4, wherein the implant main segment has a mesh structure with diamond-shaped mesh openings, which is braided from 12-32 interlaced wires, wherein 10-75 intersections per inch are formed by the wires along an axial direction.

18. The vascular implant of claim 17, wherein the implant main segment has a mesh structure with diamond-shaped mesh openings, which is braided from 16-24 interlaced wires, wherein 30-55 intersections per inch are formed by the wires along an axial direction.

19. The vascular implant of any one of claims 1 to 4, wherein a metal coverage of the implant main segment ranges from 8% to 25%.

20. The vascular implant of claim 3, wherein the cross-sectional area of the core accounts for 20%-35% of the total cross-sectional area of the wire.

21. A medical device, comprising:

the vascular implant of any one of claims 1 to 20; and
a delivery rod comprising a second joint portion; wherein the second joint portion is configured for detachable snap engagement with the first joint portion of the vascular implant.

22. The medical device of claim 21, further comprising a lumen for receiving the delivery rod and a second end of the vascular implant, wherein: when the second end of the vascular implant and the delivery rod are received within the lumen, the second end of the vascular implant is constrained by the lumen and therefore remains in snap engagement with the second joint portion, thereby axially locking the vascular implant to the delivery rod; or when the second end of the vascular implant is released from the lumen, the second end of the vascular implant is no longer constrained by the lumen and therefore detachable from the second joint portion, thereby axially unlocking the vascular implant from the delivery rod.

Fig. 1

Fig. 2

Fig. 3

Fig. 4

420

424

422

Fig. 5

122    130

Fig. 6A

122    132

126

134

Fig. 6B

122    132

126

134

Fig. 6. C

Fig. 7A

Fig. 7B

Fig. 7C

Fig. 7D

Fig. 8

## INTERNATIONAL SEARCH REPORT

| International application No. |
| --- |
| **PCT/CN2021/142230** |

### A. CLASSIFICATION OF SUBJECT MATTER

A61B 17/12(2006.01)i; A61F 2/90(2013.01)i; A61F 2/97(2013.01)i; A61F 2/966(2013.01)i

According to International Patent Classification (IPC) or to both national classification and IPC

### B. FIELDS SEARCHED

Minimum documentation searched (classification system followed by classification symbols)

A61F2/-; A61B17/-

Documentation searched other than minimum documentation to the extent that such documents are included in the fields searched

Electronic data base consulted during the international search (name of data base and, where practicable, search terms used)

WPI, EPODOC, CNPAT, CNKI: 微创, 赵涵饴, 刘子昂, 张淼, 田浩, 王芹芬, 支架, 植入, 移植, 端, 环, 编织, 连接, 输送, 递送, 芯, stent, bracket, implant, prosthe+, end, terminal, loop?, ring?, woven, braid+, knit+, weav+, connect+, deliver+, convey+, core

### C. DOCUMENTS CONSIDERED TO BE RELEVANT

| Category* | Citation of document, with indication, where appropriate, of the relevant passages | Relevant to claim No. |
| --- | --- | --- |
| PX | CN 112754584 A (SHANGHAI MICROPORT EP MEDTECH CO., LTD.) 07 May 2021 (2021-05-07)<br>claims 1-22, description paragraphs [0005]-[0104], figures 1-8 | 1-22 |
| PX | CN 113288314 A (SHANGHAI MICROPORT EP MEDTECH CO., LTD.) 24 August 2021 (2021-08-24)<br>claims 1-21, description paragraphs [0005]-[0104], figures 1-8 | 1-22 |
| PX | CN 113633433 A (SHANGHAI MICROPORT EP MEDTECH CO., LTD.) 12 November 2021 (2021-11-12)<br>description paragraphs, figure 1 | 1-3, 11-20 |
| E | CN 215874793 U (SHANGHAI MICROPORT EP MEDTECH CO., LTD.) 22 February 2022 (2022-02-22)<br>claims 1-22, description paragraphs [0005]-[0104], figures 1-8 | 1-22 |
| Y | EP 2762622 A2 (ACANDIS G.M.B.H. & CO. KG.) 06 August 2014 (2014-08-06)<br>description, paragraphs [0023]-[0037], and figures 1-4 | 1-22 |
| Y | CN 110151368 A (SHANGHAI MICROPORT EP MEDTECH CO., LTD.) 23 August 2019 (2019-08-23)<br>description, paragraphs [0085]-[0103], and figures 1-5 | 1-22 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 March 2022** | **23 March 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/142230** |

**C. DOCUMENTS CONSIDERED TO BE RELEVANT**

| Category* | Citation of document, with indication, where appropriate, of the relevant passages | Relevant to claim No. |
| --- | --- | --- |
| A | WO 2020126721 A1 (ACANDIS G.M.B.H.) 25 June 2020 (2020-06-25) entire document | 1-22 |
| A | CN 210056359 U (ACANDIS GMBH) 14 February 2020 (2020-02-14) entire document | 1-22 |
| A | CN 108260342 A (MICROVENTION INC.) 06 July 2018 (2018-07-06) entire document | 1-22 |
| A | CN 104487024 A (MICROVENTION INC.) 01 April 2015 (2015-04-01) entire document | 1-22 |

Form PCT/ISA/210 (second sheet) (January 2015)

**INTERNATIONAL SEARCH REPORT**
Information on patent family members

| International application No. |
| --- |
| **PCT/CN2021/142230** |

| Patent document cited in search report | | | Publication date (day/month/year) | Patent family member(s) | | | Publication date (day/month/year) |
| --- | --- | --- | --- | --- | --- | --- | --- |
| CN | 112754584 | A | 07 May 2021 | None | | | |
| CN | 113288314 | A | 24 August 2021 | None | | | |
| CN | 113633433 | A | 12 November 2021 | None | | | |
| CN | 215874793 | U | 22 February 2022 | None | | | |
| EP | 2762622 | A2 | 06 August 2014 | DE | 102013100984 | A1 | 14 August 2014 |
| CN | 110151368 | A | 23 August 2019 | WO | 2020259640 | A1 | 30 December 2020 |
| | | | | CA | 3143858 | A1 | 30 December 2020 |
| | | | | CN | 210749680 | U | 16 June 2020 |
| WO | 2020126721 | A1 | 25 June 2020 | EP | 3876875 | A1 | 15 September 2021 |
| | | | | DE | 102018133285 | A1 | 25 June 2020 |
| | | | | CN | 113453647 | A | 28 September 2021 |
| CN | 210056359 | U | 14 February 2020 | DE | 102018125983 | A1 | 25 April 2019 |
| CN | 108260342 | A | 06 July 2018 | JP | 2018531664 | A | 01 November 2018 |
| | | | | JP | 6816126 | B2 | 20 January 2021 |
| | | | | US | 2017079819 | A1 | 23 March 2017 |
| | | | | US | 10182931 | B2 | 22 January 2019 |
| | | | | US | 2021220158 | A1 | 22 July 2021 |
| | | | | WO | 2017049312 | A1 | 23 March 2017 |
| | | | | EP | 3349670 | A1 | 25 July 2018 |
| | | | | EP | 3349670 | B1 | 09 September 2020 |
| | | | | US | 2019142617 | A1 | 16 May 2019 |
| | | | | US | 11000394 | B2 | 11 May 2021 |
| | | | | CN | 108260342 | B | 30 July 2021 |
| CN | 104487024 | A | 01 April 2015 | WO | 2013138789 | A1 | 19 September 2013 |
| | | | | JP | 2021035510 | A | 04 March 2021 |
| | | | | US | 2017172766 | A1 | 22 June 2017 |
| | | | | US | 10543113 | B2 | 28 January 2020 |
| | | | | JP | 2015513931 | A | 18 May 2015 |
| | | | | JP | 6262712 | B2 | 17 January 2018 |
| | | | | CA | 2867181 | A1 | 19 September 2013 |
| | | | | CA | 2867181 | C | 11 August 2020 |
| | | | | US | 2019269533 | A1 | 05 September 2019 |
| | | | | US | 10765540 | B2 | 08 September 2020 |
| | | | | DE | 202013012692 | U1 | 30 July 2018 |
| | | | | JP | 2018069092 | A | 10 May 2018 |
| | | | | JP | 6830054 | B2 | 17 February 2021 |
| | | | | AU | 2013231845 | A1 | 02 October 2014 |
| | | | | AU | 2013231845 | B2 | 06 July 2017 |
| | | | | US | 2013245745 | A1 | 19 September 2013 |
| | | | | US | 9439791 | B2 | 13 September 2016 |
| | | | | EP | 2825129 | A1 | 21 January 2015 |
| | | | | EP | 2825129 | B1 | 18 January 2017 |
| | | | | EP | 2825129 | B2 | 19 February 2020 |
| | | | | EP | 3156006 | A1 | 19 April 2017 |
| | | | | US | 2020368049 | A1 | 26 November 2020 |
| | | | | US | 2016361180 | A1 | 15 December 2016 |
| | | | | US | 10335297 | B2 | 02 July 2019 |
| | | | | KR | 20140140077 | A | 08 December 2014 |
| | | | | KR | 101871144 | B1 | 27 June 2018 |

Form PCT/ISA/210 (patent family annex) (January 2015)

**INTERNATIONAL SEARCH REPORT**
Information on patent family members

| Patent document cited in search report | Publication date (day/month/year) | Patent family member(s) | | Publication date (day/month/year) |
|---|---|---|---|---|
| | | KR  20180071414 | A | 27 June 2018 |
| | | KR  101968885 | B1 | 15 April 2019 |
| | | CN  104487024 | B | 29 August 2017 |

International application No.

**PCT/CN2021/142230**

Form PCT/ISA/210 (patent family annex) (January 2015)